# EUROPÄISCHE PATENTSCHRIFT

(11) **EP 3 205 221 B1**
(45) Veröffentlichungstag und Bekanntmachung des Hinweises auf die Patenterteilung: **14.03.2018**
(21) Anmeldenummer: 17160741.9
(22) Anmeldetag: 27.12.2010
(51) Int. Cl.: A41D 13/12

(54) **ORTHESE**
ORTHOSIS
ORTHÈSE

(30) Priorität: 30.12.2009 DE 202009017677 U
(43) Veröffentlichungstag der Anmeldung: 16.08.2017
(62) Teilanmeldung aus: 10805710.0
(73) Patentinhaber: Otto Bock HealthCare GmbH, 37115 Duderstadt (DE)
(72) Erfinder: SCHODDE, Helgard, 26384 Wilhelmshafen (DE)
(74) Vertreter: Gramm, Lins & Partner Patent- und Rechtsanwälte PartGmbB

(56) Entgegenhaltungen:
- WO-A1-95/19154
- DE-U1-202008 002 836
- US-A- 4 607 629
- US-A- 5 123 407
- US-A- 5 377 693
- US-A- 5 895 366
- US-A1- 2006 074 365

## Beschreibung

Die Erfindung betrifft eine orthopädische Prothese, kurz Orthese, insbesondere für Menschen mit spastischer Cerebralparese, mit mindestens einem im Beinbereich positionierbaren Stützelement zum Abstützen, insbesondere Spreizen eines Beines oder beider Beine, und mindestens einer Halteeinrichtung zum Halten des mindestens ein Stützelementes, welche derart mit dem Stützelement gekoppelt ist, das beim Tragen der Orthese das Stützelement im Wesentlichen in einer Sollposition gehalten werden kann.

Eine derartige Orthese ist beispielsweise aus der US 4,607,629 A bekannt.

Menschen, die von Cerebralparese betroffen sind, leiden unter Bewegungsstörungen, die durch Schädigungen im sensomotorischen System verursacht werden. Das sensomotorische System ist bei Menschen aus einem geschlossenen Regelkreis zwischen dem pyramidalen System, das die Gehirnregionen (Cerebrum) für Bewegungssteuerungen umfasst, und dem extrapyramidal motorischen System aufgebaut, unter der die Nervenbahnen zur Übertragung von Bewegungsimpulsen verstanden werden. Das extrapyramidal motorische System empfängt ein Bewegungssignal aus dem pyramidalen System, worauf die Bewegung ausgeführt wird und anschließend eine Rückmeldung an das pyramidale System erfolgt, so dass ein geschlossener Regelkreis entsteht. Von spastischer Cerebralparese betroffene Menschen weisen in der Regel Schädigungen in beiden Teilen des sensomotorischen Regelkreises auf, wodurch die Bewegungskontrolle zeitweise aussetzt. Die Symptome zeigen sich durch hohe Muskelspannungen (Muskelhypertonie) oder ständiges Wechseln von starken und schwachen Muskelspannungen (Muskelhypotonie), die durch pathologische Körperhaltungen ausgeglichen werden. Eines davon ist die Fehlstellung der Beine beim Stehen oder Gehen, die sich durch stark nach innen gebeugte Beine auszeichnet.

Bereits bekannt sind Metallschienengestelle, die außen am Bein befestigt werden, um die Fehlstellung der Beine zu korrigieren. Diese Vorrichtungen haben den Nachteil, dass sie starr sind und somit die Bewegung des Anwenders stark einschränken. Darüber hinaus sind diese Vorrichtungen schwer und unästhetisch, wodurch der Komfort, Wohlbefinden und die soziale Akzeptanz des Anwenders beeinträchtigt werden.

Bekannt ist auch der Einsatz von orthopädischen Fußsohleneinlagen zur positiven Beeinflussung der Körperhaltung. Sie weisen den Nachteil auf, dass sich die Körperhaltung nur unwesentlich verbessert und für Menschen mit ausgeprägten Fehlstellungen ungeeignet sind.

Aus der US 5,377,693 A ist eine Hose bekannt, bei der an der Innenseite der Hosenbeine Polster angeordnet sind, die als Abstandshalter fungieren. Diese Polster befinden sich in auf die Hose aufgesetzten Taschen.

Die US 5,123,407 A offenbart eine kurze Hose, die an den Oberschenkelinnenseiten Taschen aufweist, in die keilförmige Polster als Abstandshalter einschiebbar sind.

Aus der DE 20 2008 002 836 U1 ist ein Kissen zur Beinfixierung bekannt, das an einem Bein befestigt wird und für eine gepolsterte Zwischenlage zwischen den Beinen sorgt. Dazu wird das andere Bein relativ zum ersten Bein fixiert.

Die US 5,895,366 A beschreibt eine Vorrichtung, bei der ein einheitliches Kissen als Brückenelement zwischen den beiden Schenkeln befestigbar ist. Es handelt sich um ein mit Klettverschlüssen fixierbares Fluidkissen, das nach Bedarf aufgepumpt werden kann.

Aus der WO 95/19154 A1 ist ein Hüftprotektor bekannt, der in ein Unterwäsche-Kleidungsstück einsetzbar ist und die Außenseite des jeweiligen Hüftgelenkes schützt. Aus der US 2006/0074365 A1 ist eine Hüftstützvorrichtung bekannt, bei der zwischen einem Hüftgurt und einem oberhalb des Knies am Oberschenkel befestigten Gurt vertikal Gurte angeordnet sind.

Aufgabe der Erfindung ist es daher eine Orthese, insbesondere für Menschen mit spastischer Cerebralparese und einer X-Bein Fehlstellung, anzugeben, welche die Bewegungsfreiheit des Anwenders wenig einschränkt und alltagstauglich ist.

Erfindungsgemäß wird die Aufgabe durch eine Orthese nach Anspruch 1 gelöst, die sich dadurch auszeichnet, dass die Halteeinrichtung mindestens einen verstellbaren Gurt enthält wobei mindestens ein Ende mit dem Stützelement gekoppelt ist, und dadurch, dass die Halteeinrichtung einen um den Bauchbereich legbaren Tragegurt aufweist und jedes Stützelement mittels mindestens einem weiteren Haltegurt gehalten wird. Erfindungsgemäß wird die Aufgabe ferner durch eine Orthese nach Anspruch 2 gelöst, die sich dadurch auszeichnet, dass die Halteeinrichtung eine Kombination aus Gurten und einer Hose mit Taschen zur Aufnahme eines Stützelements ist.

Durch die erfindungsgemäße Orthese wird auf besonders einfache Weise erreicht, dass die Beine des Anwenders beim Gehen und Stehen annähernd die Normalstellung einnehmen. Allein durch die Positionierung eines Stützelements zwischen die Beine wird das Einknicken der Beine nach innen erfolgreich verhindert, indem die Beine abgestützt bzw. gespreizt werden. Durch die Verbesserung der Beinstellung wird gleichzeitig auch eine bessere Fußauflage, Fußgelenk- und Hüftstellung erreicht, so dass Kontrakturen des Anwenders korrigiert werden und ihnen prophylaktisch entgegengewirkt wird.

Insbesondere durch die Kopplung mit einer Halteeinrichtung verbleibt das Stützelement auch beim Gehen oder vergleichbaren Bewegungen in einer gewünschten Sollposition, die sich je nach Anwender insbesondere im Bereich zwischen Knie und unterem Hüftansatz befindet. Der Anwender kann somit seine körperlichen Tätigkeiten ausführen, ohne die Orthese immer wieder nachjustieren zu müssen. Somit wird zu jedem Zeitpunkt die Stützfunktion durch die Orthese gewährleistet mit dem Ergebnis, dass die Bewegungsfreiheit und der Komfort des Anwenders durch die Orthese wenig eingeschränkt wird. Ferner ist die Orthese so kompakt, dass es unter alltäglichen Kleidungsstücken getragen werden kann, ohne von außen signifikant aufzufallen.

Die Stützfunktion und die Korrektur der Fehlstellung der Beine wird besonders effektiv dadurch erreicht, dass die erfindungsgemäße Orthese mit zwei Stützelementen ausgestattet wird, die jeweils einem Bein zugeordnet und mittels der Halteeinrichtung beim Tragen an der Innenseite des jeweiligen Beines angeordnet sind. Die Dicke der Stützelemente kann gegenüber einer Orthese mit nur einem Stützelement ohne Verlust der Spreizkraft um etwa die Hälfte verringert werden. Die Spreizkraft resultiert als Reaktionskraft aus dem Druck beim Angrenzen zweier Stützelemente an der Innenseite der Beine.

Mittels einer bevorzugten erfindungsgemäßen Orthese mit einem gekrümmt geformten Stützelement, das einen Bereich am inneren Oberschenkel eines Beines teilweise umgreift, erreicht man eine besonders zuverlässige Einhaltung der Sollposition am Beinbereich des Anwenders. Zusätzlich dazu stellt sich beim Anlegen eines solch geformten Stützelements durch die gleichmäßigere Druckverteilung auf eine umfassende Beinoberfläche ein angenehmer haptischer Eindruck für den Anwender ein.

Eine erfindungsgemäße Orthese, deren Stützelement so geformt ist, dass das Stützelement genau am Innenschenkelbereich anliegt, erreicht einen besonders angenehmen haptischen Eindruck beim Anwender. Ferner erzielt diese Eigenschaft auch den Vorteil eines erhöhten Tragekomforts, indem mögliche Reibungen zwischen dem Innenschenkelbereich und dem Stützelement minimiert werden.

Mit einer erfindungsgemäßen Orthese, deren Außenseite des Stützelementes so geformt ist, dass sie an das Ausmaß der Fehlstellung der Beine angepasst ist, erreicht auf eine äußerst präzise Art und Weise, dass die nach außen wirkende Spreizkraft an die Bedürfnisse des Anwenders angepasst sind. Das Profil der Außenseite kann in zwei Achsen variiert werden. Einerseits ergibt sich aus dem Profil der Außenseite entlang des Umfangs die Größe der Spreizkraft auf die Beine entsprechend ihrer relativen Stellung beim Gehen. Das Profil der Außenseite in vertikaler Richtung, d. h. von der Hüfte zum Knie verlaufend, definiert die Spreizkraft nach der Ausprägung der Beinanatomie des Anwenders. Die Spreizkraft ist also auf die alltäglichen Anwendungssituation und anatomischen Gegebenheiten des Anwenders abgestimmt.

Mit einer erfindungsgemäßen Orthese, deren Stützelemente an den Enden steifer sind als die mittleren Bereiche, erreicht man auf eine besonders zuverlässige Weise eine höhere Lebensdauer des Stützelements.

Die erfindungsgemäße Orthese mit einer Halteeinrichtung, die mindestens einen verstellbaren Gurt enthält, wobei mindestens ein Ende mit dem Stützelement gekoppelt ist, erhöht auf besonders einfache Art und Weise die individuelle Anpassbarkeit der Orthese je nach den Bedürfnissen des Anwenders im Alltag und seinen anatomischen Gegebenheiten. Auf gleichem Wege wird auch eine zuverlässige Einhaltung der gewünschten Sollposition erreicht.

Eine besonders bevorzugte Ausführungsform der erfindungsgemäßen Orthese besteht darin, dass die Halteeinrichtung einen um den Bauchbereich legbaren Tragegurt aufweist und jedes Stützelement mittels mindestens eines weiteren Haltegurtes gehalten wird. Diese Ausführungsform erreicht ein besonders hervorstehendes, stabiles und zuverlässiges Anliegen der Stützelemente am Innenbereich der Beine in der gewünschten Sollposition.

Bevorzugt ist weiterhin eine Ausführungsform, bei der der Tragegurt und/oder der Haltegurt einen Klettverschluss aufweist, mit dem die Länge variierbar ist. Dadurch wird dem Anwender auf besonders einfache Art und Weise ermöglicht, dass er die Halteeinrichtung jederzeit mit einfachen Handgriffen an seine Bedürfnisse anpassen kann.

Mit einer erfindungsgemäßen Orthese, bei der die Halteeinrichtung eine Hose aufweist, an welcher mindestens ein Stützelement befestigt ist, wird dem Anwender eine besonders hohe Alltagstauglichkeit geboten, da die Hose in der Regel ohne Hilfe Dritter ein- und ausgezogen werden kann. Das Anlegen der Orthese gestaltet sich dadurch auch für Menschen mit körperlichen Einschränkungen als ausführbare Aufgabe.

Besonders bevorzugt ist eine Ausführungsform, bei der die Hose eine Tasche aufweist, in welcher ein Stützelement einlegbar ist. Zu den bereits genannten Vorteilen, erreicht die Hose mit einer Tasche den besonders guten Schutz des Stützelements vor Schmutz und Schweiß in der Anwendung und eine gute Positionierung des Stützelementes.

Bevorzugt ist weiterhin eine Ausführungsform, bei der die Hose nach Art einer kurzen Radlerhose ausgebildet ist, wobei die Taschen im Innenbereich ausgebildet sind. Neben der Alltagstauglichkeit, bietet diese Ausführungsform dem Anwender eine höhere Akzeptanz in seinem sozialen Umfeld. Die Hose verdeckt die Stützelemente, so dass sie von außen nicht sichtbar und auf dem ersten Blick nicht als Orthese erkennbar sind. Der Anwender hat damit auch die Möglichkeit, die Orthese beispielsweise in privaten Räumen ohne ein weiteres Kleidungsstück oder unter einem solchen tragen zu können.

Vorteilhaft ist auch eine erfindungsgemäße Orthese, bei der die Halteeinrichtung eine Kombination aus Gurten und eine Hose mit Taschen zur Aufnahme eines Stützelements ist. Die Anordnung eines Stützelementes in einer Tasche der Hose und zusätzliche Gurte, die mit dem Stützelement gekoppelt sind, erlauben eine besonders zuverlässige Haltung der Stützelemente an ihrer gewünschten Sollposition und ein festes Anliegen an den Beinen des Anwenders.

Die erfindungsgemäße Orthese mit einem Stützelement aus einem Schaumstoff bietet dem Anwender durch seine dämpfenden Eigenschaften einen besonders hohen Tragekomfort bei gleichzeitiger Erfüllung der Stützfunktion.

Eine besonders bevorzugte Ausführungsform der erfindungsgemäßen Orthese besteht darin, dass der Schaumstoff ausgewählt ist aus der Gruppe bestehend aus den Materialen nach Anspruch 16, wodurch auf besonders einfache Art und Weise ein Schaumstoff hergestellt werden kann, der in seiner Elastizität, Härtegrad und Langlebigkeit den individuellen Bedürfnissen des Anwenders entspricht.

Eine erfindungsgemäße Orthese bei der die Hose ausgewählt ist aus einer Gruppe von Textilfasern nach Anspruch 17 weist die besonders vorteilhaften Eigenschaften auf, elastisch, hautfreundlich und atmungsaktiv zu sein.

Ein Ausführungsbeispiel der Erfindung ist in den folgenden Zeichnungen dargestellt und wird im Folgenden näher erläutert. Es zeigt:
- Figur 1: eine Frontansicht der Orthese im getragenen Zustand;
- Figur 2: eine Rückansicht der Orthese im getragenen Zustand;
- Figur 3: eine Schnittansicht von oben entlang der Linie A-A.

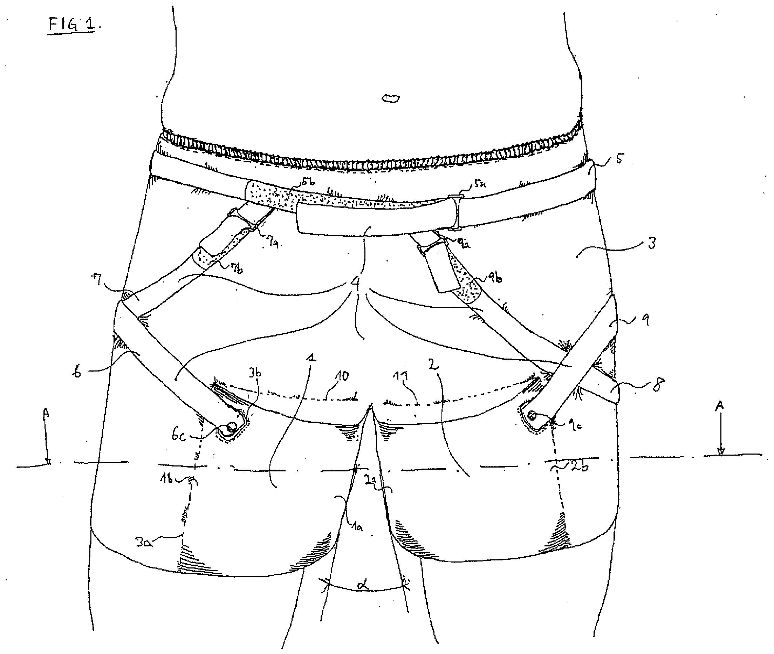

Figur 1 zeigt eine Orthese mit zwei im Beinbereich positionierten Stützelementen 1, 2 zum Abstützen insbesondere Spreizen der Beine und einer Halteeinrichtung 4 zum Halten der Stützelemente 1, 2, so dass beim Tragen der Orthese die Stützelemente 1, 2 im Wesentlichen in einer Sollposition gehalten werden können. Alternativ kann die Orthese auch mit nur einem Stützelement ausgebildet sein (nicht dargestellt). Jedes Stützelement 1, 2 ist jeweils einem Bein zugordnet und an der Innenseite des jeweiligen Beines so angeordnet, dass eine nach außen gerichtete Spreizkraft auf die Beine ausgeübt wird. Die Stützelemente 1, 2 sind dabei so gekrümmt geformt, dass sie einen Bereich am inneren Oberschenkel eines Beines umgreifen und im mittleren Bereich 1a, 2a eine größere Dicke aufweisen, als an den Randbereichen 1b, 2b. An den Randbereichen 1b, 2b sind die Stützelemente 1, 2 steifer als die mittleren Bereiche 1a, 2a, wodurch die Kopplung 6c, 7c, 8c, 9c zwischen den Haltegurten 6, 7, 8, 9 und Stützelementen 1, 2 stabilisiert wird.

In der hier gezeigten Ausführung ist die Halteeinrichtung 4 eine Kombination aus mehreren Gurten 5, 6, 7, 8, 9 und einer Hose 3 mit im Innenbereich ausgebildeten Taschen 10, 11. Die Gurte 5, 6, 7, 8, 9 umfassen einen um den Bauchbereich legbaren Tragegurt 5, an dem weitere Haltegurte 6, 7, 8, 9 angenäht sind. Die Gurte 6, 7, 8, 9, sind an einem Ende mit den Stützelementen 1, 2 gekoppelt. Die Gurte 6c, 7c, 8c, 9c und sorgen beim Tragen der Orthese dafür, dass die Stützelemente 1, 2 im Wesentlichen in einer Sollposition gehalten werden. Zudem weisen die Gurte 5, 6, 7, 8, 9 einen Klettverschluss 5b, 6b, 7b, 8b, 9b auf, mit dem die Länge verstellbar ist. Dies ermöglicht eine einfache Anpassbarkeit an einen Benutzer und auch, die auf die Stützelemente ausgeübte Zugkraft zu variieren. Ferner enthält die Hose 3 an den Kopplungen 6c, 7c, 8c, 9c Löcher 3b, die mit einer umlaufenden Naht versehen sind.

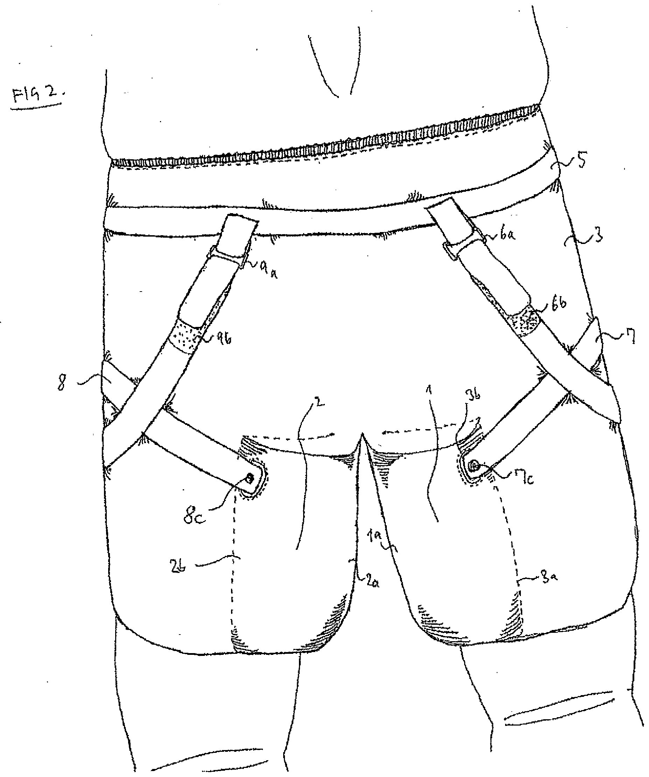

Figur 2 zeigt den weiteren Verlauf der Stützelemente 1, 2 und Gurte 5, 6, 7, 8, 9 auf der Rückseite der Hose. Jeweils zwei Gurte 6, 7; 8, 9 verlaufen überkreuzt von vorne am Traggurt 5 nach hinten zum Stützelement 1, 2 bzw. von hinten am Traggurt 5 nach vorne zum Stützelement 1, 2. Werden die Gurte über die Klettverschlüsse 6b, 7b, 8b, 9b verkürzt und dadurch gespannt, erfahren die Stützelemente an beiden Randbereichen 1 b, 2b eine Zugkraft diagonal nach oben, so dass sie stärker an die Innenschenkel gedrückt werden und noch besser gegen Verrutschen abgesichert sind.

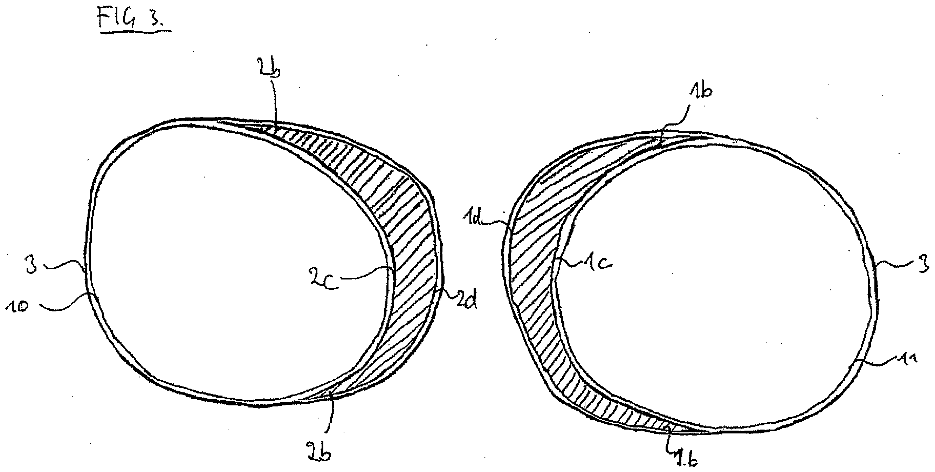

Figur 3 zeigt einen Querschnitt aus dem Beinbereich der Orthese mit der Hose 3 und mit im Innenbereich ausgebildeten Taschen 10, 11. Die Hose 3 ist nach Art einer kurzen Radlerhose ausgebildet und besitzt im Innenbereich angenähte Taschen 10, 11, in der die Stützelemente 1, 2 eingelegt werden. Die Taschen 10, 11 und Hose 3 liegen eng und elastisch an den Stützelementen 1, 2 und tragen zur weiteren Befestigung und Positionierung der Stützelemente 1, 2 in einer gewünschten Position bei. Die Innenseiten 1c, 2c der Stützelemente 1, 2 sind so geformt, dass die Stützelemente 1, 2 möglichst passgenau am Innenschenkelbereich anliegen. Die Außenseiten 1d, 2d der Stützelemente 1, 2 haben derweil eine Form, die an das Ausmaß der Fehlstellung der Beine angepasst ist. Je nach Fehlstellung ist der Winkel α zwischen den Beinen unterschiedlich. Die Spreizkraft auf die Beine des Anwenders vergrößert sich, je kleiner der Winkel α zwischen den Stützelementen 1, 2 wird und die Außenseiten 1d, 2d der Stützelemente gegeneinander gedrückt werden. Durch die genaue Form und Dicke der Stützelemente 1, 2 kann eine Anpassung an einen Benutzer und individuelle Anpassung einer Spreizkraft vorgenommen werden.

Eine Orthese, insbesondere für Menschen mit spastischer Cerebralparese, kann mindestens ein im Beinbereich positionierbares Stützelement zum Abstützen insbesondere Spreizen meines Beines oder beider Beine und mindestens eine Halteeinrichtung zum Halten des mindestens einen Stützelementes aufweisen, welches derart mit dem Stützelement gekoppelt ist, dass beim Tragen der Orthese das mindestens eine Stützelement im Wesentlichen in einer Sollposition gehalten werden kann.

Die Orthese nach dem vorangegangenen Absatz zeichnet sich vorzugsweise dadurch aus, dass zwei Stützelemente vorgesehen sind, die jeweils einem Bein zugeordnet sind und mittels der Halteeinrichtung beim Tragen an der Innenseite des jeweiligen Beines so angeordnet sind, dass eine nach außen gerichtete Spreizkraft auf die Beine ausgeübt wird.

Die Orthese nach dem vorangegangenen Absatz zeichnet sich vorzugsweise dadurch aus, dass ein Stützelement so gekrümmt geformt ist, dass es einen Bereich am inneren Oberschenkel eines Beines teilweise umgreift.

Eine Orthese nach einem der drei vorstehenden Absätze zeichnet sich vorzugsweise dadurch aus, dass das Stützelement in einem mittleren Bereich eine größere Dicke aufweist als in einem Randbereich.

Die Orthese nach mindestens einem der vorstehenden Abätze zeichnet sich vorzugsweise dadurch aus, dass eine Innenseite des Stützelements so geformt ist, dass das Stützelement passgenau am Innenschenkelbreich anliegt.

Die Orthese nach mindestens einem der vorstehenden Abätze zeichnet sich vorzugsweise dadurch aus, dass eine Außenseite des Stützelements so geformt ist, dass sie an das Ausmaß der Fehlstellung der Beine angepasst ist.

Die Orthese nach einem der vorstehenden Abätze zeichnet sich vorzugsweise dadurch aus, dass die Randbereiche des Stützelements steifer sind als die mittleren Bereiche.

Die Orthese nach einem der vorstehenden Abätze zeichnet sich vorzugsweise dadurch aus, dass die Halteeinrichtung mindestens einen verstellbaren Gurt enthält, wobei mindestens ein Ende mit dem Stützelement 1,2 gekoppelt ist.

Die Orthese nach dem vorangegangenen Absatz zeichnet sich vorzugsweise dadurch aus, dass die Halteeinrichtung einen um den Bauchbereich legbaren Trage-Gurt aufweist und jedes Stützelement mittels mindestens eines weiteren Halte-Gurtes gehalten wird.

Die Orthese nach einem der beiden vorangegangenen Absätze zeichnet sich vorzugsweise dadurch aus, dass der Trage-Gurt einen Klettverschluss aufweist, mit dem die Länge variierbar ist.

Die Orthese nach mindestens einem der vorstehenden Abätze zeichnet sich vorzugsweise dadurch aus, dass die Halteeinrichtung eine Hose aufweist, an welcher mindestens ein Stützelement befestigbar ist.

Die Orthese nach dem vorangegangenen Absatz zeichnet sich vorzugsweise dadurch aus, dass die Hose eine Tasche aufweist, in welche ein Stützelement einlegbar ist.

Die Orthese nach einem der beiden vorangegangenen Absätze zeichnet sich vorzugsweise dadurch aus, dass die Hose nach Art einer kurzen Radlerhose ausgebildet ist, wobei die Taschen im Innenbereich ausgebildet sind.

Die Orthese nach einem der vorstehenden Abätze zeichnet sich vorzugsweise dadurch aus, dass die Halteeinrichtung eine Kombination aus Gurten und einer Hose mit Taschen zur Aufnahme eines Stützelementes ist.

Die Orthese nach mindestens einem der vorstehenden Abätze zeichnet sich vorzugsweise dadurch aus, dass ein Stützelement aus einem Schaumstoff gebildet ist.

Die Orthese nach dem vorangegangenen Absatz zeichnet sich vorzugsweise dadurch aus, dass der Schaumstoff ausgewählt ist aus der Gruppe bestehend aus den Materialien Formaldehyd-Harze, Polyisocyanurate, Polystyrol, Polyurethane, Polyvinylchlorid, Ethylenvinylacetat und Mischungen davon.

Die Orthese nach einem der vorstehenden Abätze zeichnet sich vorzugsweise dadurch aus, dass die Hose ausgewählt ist aus der Gruppe bestehend aus den Textilfasern Baumwolle, Cupro, Lyocell, Modalfasern, Viskosefasern, Acetatfasern, Triacetatfasern, Alginatfasern, Polyacrylfasern, Aramidfasern, Polychloridfasern, Elasthan, Elastodien, Flurofasern, Modacrylfasern, Polyamidfasern, Polyesterfasern, Polyethylenfasern, Polyimidfasern, Polypropylenfasern, Polyesterfasern, Polyethylenfasern, Polyimidfasern, Polypropylenfasern, Vinylfasern und Mischungen davon.

## Patentansprüche

1. Orthese, insbesondere für Menschen mit spastischer Cerebralparese, mit
- mindestens einem an der Innenseite des Oberschenkels positionierbaren Stützelement (1, 2) zum Abstützen, insbesondere Spreizen, eines Beines oder beider Beine und
- mindestens einer Halteeinrichtung (4) zum Halten des mindestens einen Stützelements (1, 2), **dadurch gekennzeichnet, dass** die Halteeinrichtung (4) einen um den Bauchbereich legbaren Tragegurt (5) aufweist,
mindestens einen verstellbaren Gurt (6, 8) enthält, dessen erstes Ende mit einem vorderen Bereich (6c) des mindestens einen Stützelementes (1, 2) und dessen zweites Ende mit einem hinteren Bereich des Tragegurtes gekoppelt ist, und mindestens einen weiteren Haltegurt (7, 9) enthält, dessen erstes Ende mit einem hinteren Bereich (7c) des mindestens einen Stützelementes (1, 2) und dessen zweites Endet mit einem vorderen Bereich des Tragegurtes gekoppelt ist,
so dass beim Tragen der Orthese das mindestens eine Stützelement (1, 2) im Wesentlichen in einer Sollposition gehalten werden kann.

2. Orthese nach Anspruch 1, **dadurch gekennzeichnet, dass** die Halteeinrichtung (4) eine Kombination aus Gurten (5, 6, 7, 8, 9) und einer Hose (3) mit Taschen (10, 11) zur Aufnahme eines Stützelements (1, 2) ist.

3. Orthese nach Anspruch 1 oder 2, **dadurch gekennzeichnet, dass** zwei Stützelemente (1, 2) vorgesehen sind, die jeweils einem Bein zugeordnet sind und mittels der Halteeinrichtung (4) beim Tragen an der Innenseite des jeweiligen Beines so angeordnet sind, dass eine nach außen gerichtete Spreizkraft auf die Beine ausgeübt wird.

4. Orthese nach Anspruch 3, **dadurch gekennzeichnet, dass** ein Stützelement (1, 2) so gekrümmt geformt ist, dass es einen Bereich am inneren Oberschenkel eines Beines teilweise umgreift.

5. Orthese nach Anspruch 1, 2, 3 oder 4, **dadurch gekennzeichnet, dass** das Stützelement (1, 2) in einem mittleren Bereich (1a, 2a) eine größere Dicke aufweist als in einem Randbereich (1b, 2b).

6. Orthese nach mindestens einem der vorstehenden Ansprüche, **dadurch gekennzeichnet, dass** eine Innenseite des Stützelements (1, 2) so geformt ist, dass das Stützelement (1, 2) passgenau am Innenschenkelbereich anliegt.

7. Orthese nach einem der vorstehenden Ansprüche, **dadurch gekennzeichnet, dass** die Randbereiche (1 b, 2b) des Stützelements (1, 2) steifer sind als die mittleren Bereiche (1a, 2a).

8. Orthese nach einem der vorstehenden Ansprüche, **dadurch gekennzeichnet, dass** der Trage-Gurt (5) einen Klettverschluss (5b) aufweist, mit dem die Länge variierbar ist.

9. Orthese nach mindestens einem der vorstehenden Ansprüche, **dadurch gekennzeichnet, dass** ein Stützelement (1, 2) aus einem Schaumstoff gebildet ist.

10. Orthese nach Anspruch 9, **dadurch gekennzeichnet, dass** der Schaumstoff ausgewählt ist aus der Gruppe bestehend aus den Materialien FormaldehydHarze, Polyisocyanurate, Polystyrol, Polyurethane, Polyvinylchlorid, Ethylenvinylacetat und Mischungen davon.

11. Orthese nach einem der vorstehenden Ansprüche 2 bis 10, **dadurch gekennzeichnet, dass** die Hose ausgewählt ist aus der Gruppe bestehend aus den Textilfasern Baumwolle, Cupro, Lyocell, Modalfasern, Viskosefasern, Acetatfasern, Triacetatfasern, Alginatfasern, Polyacrylfasern, Aramidfasern, Polychloridfasern, Elasthan, Elastodien, Fluorofasern, Modacrylfasern, Polyamidfasern, Polyesterfasern, Polyethylenfasern, Polyimidfasern, Polypropylenfasern, Vinylalfasern und Mischungen davon.

## Claims

1. An orthotic, in particular for people who have spastic cerebral palsy, having:
- at least one support element (1, 2) positionable on the inside of the thigh for supporting, in particular spreading, one leg or both legs, and,
- at least one holding device (4) for holding the at least one support element (1, 2), **characterized in that** the holding device (4) has a harness (5) that may be placed about the abdominal region,
includes at least one adjustable belt (6, 8), the first end of which is coupled to a front region (6c) of the at least one support element (1, 2) and the second end of which is coupled to a rear region of the harness, and includes at least one other tether (7, 9), the first end of which is coupled to a rear region (7c) of the at least one support element (1, 2) and the second end of which is coupled to a front region of the harness,
so that when the orthotic is worn the at least one support element (1, 2) may be held essentially in a target position.

2. The orthotic according to claim 1, **characterized in that** the holding device (4) is a combination of belts (5, 6, 7, 8, 9) and a pair of pants (3) having pockets (10, 11) for receiving a support element (1, 2).

3. The orthotic according to claim 1 or 2, **characterized in that** two support elements (1, 2) are provided that are allocated to one leg each and, when worn on the inside of the respective leg, are arranged by means of the holding device (4) such that an outwardly exerted spreading force is exerted on the legs.

4. The orthotic according to claim 3, **characterized in that** a support element (1, 2) is shaped curved such that it partially grips a region on the inner thigh of a leg.

5. The orthotic according to claim 1, 2, 3, or 4, **characterized in that** the support element (1, 2) in a center region (1a, 2a) has a greater thickness than in an edge region (1b, 2b).

6. The orthotic according to at least one of the foregoing claims, **characterized in that** an inside of the support element (1, 2) is shaped such that the support element (1, 2) is positioned fitting precisely against the inner thigh region.

7. The orthotic according to any of the foregoing claims, **characterized in that** the edge regions (1b, 2b) of the support element (1, 2) are stiffer than the center regions (1a, 2a).

8. The orthotic according to any of the foregoing claims, **characterized in that** the harness (5) has a hook-and-loop fastener (5b) with which its length may be varied.

9. The orthotic according to at least one of the foregoing claims, **characterized in that** a support element (1, 2) is formed from a foam.

10. The orthotic according to claim 9, **characterized in that** the foam is selected from the group comprising the materials formaldehyde resins, polyisocyanurates, polystyrene, polyurethanes, polyvinylchloride, ethylene vinyl acetate, and mixtures thereof.

11. The orthotic according to any of the foregoing claims 2 through 10, **characterized in that** the pants are selected from the group comprising the textile fibers cotton, cupro, lyocell, modal fibers, viscose fibers, acetate fibers, triacetate fibers, alginate fibers, polyacrylic fibers, aramide fibers, polychloride fibers, elastane, elastodiene, fluoro fibers, modacrylic fibers, polyamide fibers, polyester fibers, polyethylene fibers, polyimide fibers, polypropylene fibers, vinylal fibers, and mixtures thereof.

## Revendications

1. Orthèse, destinée en particulier pour des personnes souffrant d'une parèse cérébrale spastique, comportant
- au moins un élément de soutien (1, 2) susceptible d'être positionné sur le côté intérieur de la cuisse et destiné à soutenir, en particulier à écarter, une jambe ou les deux jambes, et
- au moins un dispositif de retenue (4) pour retenir ledit au moins un élément de soutien (1, 2),
**caractérisée en ce que**
le dispositif de retenue (4)
comprend une sangle à porter (5) susceptible d'être posée autour de la zone ventrale,
présente au moins une sangle réglable (6, 8) dont la première extrémité est couplée à une zone avant (6c) dudit au moins un élément de soutien (1, 2) et dont la seconde extrémité est couplée à une zone arrière de la sangle à porter,
et comporte au moins une autre sangle de retenue (7, 9) dont la première extrémité est couplée à une zone arrière (7c) dudit au moins un élément de soutien (1, 2) et dont la seconde extrémité est couplée à une zone avant de la sangle à porter,
de sorte que lorsque l'orthèse est portée, ledit au moins un élément de soutien (1, 2) peut être maintenu sensiblement dans une position de consigne.

2. Orthèse selon la revendication 1,
**caractérisée en ce que**
le dispositif de retenue (4) est une combinaison de sangles (5, 6, 7, 8, 9) et d'un pantalon (3) ayant des poches (10, 11) pour loger un élément de soutien (1, 2).

3. Orthèse selon la revendication 1 ou 2,
**caractérisée en ce que**
il est prévu deux éléments de soutien (1, 2) qui sont associés chacun à une jambe et qui sont agencés, lorsque l'orthèse est portée, sur le côté intérieur de la jambe respective au moyen du dispositif de retenue (4), de telle sorte qu'un effort d'écartement dirigé vers l'extérieur est exercé sur les jambes.

4. Orthèse selon la revendication 3,
**caractérisée en ce que**
l'élément de soutien (1, 2) est formé incurvé de manière à entourer partiellement une zone sur la cuisse intérieure d'une jambe.

5. Orthèse selon la revendication 1, 2, 3 ou 4,
**caractérisée en ce que**
dans une zone médiane (1a, 2a), l'élément de soutien (1, 2) présente une épaisseur supérieure à celle dans une zone de bord (1b, 2b).

6. Orthèse selon l'une au moins des revendications précédentes, **caractérisée en ce que**
un côté intérieur de l'élément de soutien (1, 2) est formé de telle sorte que l'élément de soutien (1, 2) s'appuie contre la zone intérieure de la cuisse de manière exactement ajustée.

7. Orthèse selon l'une au moins des revendications précédentes, **caractérisée en ce que**
les zones de bord (1b, 2b) de l'élément de soutien (1, 2) sont plus rigides que les zones médianes (1a, 2a).

8. Orthèse selon l'une au moins des revendications précédentes, **caractérisée en ce que**
la sangle à porter (5) présente une fermeture à crochets-et-bouclettes (5b) permettant de varier la longueur.

9. Orthèse selon l'une au moins des revendications précédentes, **caractérisée en ce que**
un élément de soutien (1, 2) est formé en matière de mousse.

10. Orthèse selon la revendication 9,
**caractérisée en ce que**
la mousse est choisie parmi le groupe comprenant les matériaux suivants :
résines de formaldéhyde, polyisocyanurates, polystyrène, polyuréthanes,
chlorure de polyvinyle, éthylène-acétate de vinyle, et leurs mélanges.

11. Orthèse selon l'une des revendications 2 à 10,
**caractérisée en ce que**
le pantalon est choisi parmi le groupe comprenant les fibres textiles suivantes : coton, cupro, lyocell, fibres de modal, fibres de viscose, fibres d'acétate, fibres de tri-acétate, fibres d'alginate, fibres polyacryliques, fibres d'aramide, fibres de polychlorure, élasthanne, élastodiène, fibres fluorées, fibres modacryliques, fibres de polyamide, fibres de polyester, fibres de polyéthylène, fibres de polyimide, fibres de polypropylène, fibres de vinylal, et leurs mélanges.
